# EUROPEAN PATENT APPLICATION

(11) **EP 1 785 149 A1**
(43) Date of publication of application: **16.05.2007**
(21) Application number: 05770469.4
(22) Date of filing: 10.08.2005
(51) Int. Cl.: A61L 2/20

(54) **METHOD OF STERILIZATION AND STERILIZATION APPARATUS**

(30) Priority: 10.08.2004 JP 2004233781
(71) Applicant: Bio Media Co. Ltd., Chiyoda-ku, Tokyo 1010021 (JP); Hashiba, Tomohiko, Chiyoda-ku, Tokyo 1010021 (JP)
(72) Inventor: HASHIBA, Tomohiko c/o Bio Media Co., Ltd, Chiyoda-ku Tokyo 1010021; (JP); KAWAMURA, Koji, Ishikawa-gun Ishikawa 9218845 (JP)
(74) Representative: Epping - Hermann - Fischer
(86) International application number: PCT/JP2005/014686
(87) International publication number: WO 2006/016620

(57) **Abstract**

Highly versatile method of sterilization and sterilization apparatus, in which sterilization subjects of poor heat resistance, such as plastic products, can be sterilized without incurring thermal damages with such a high sterilization power that spores can be killed. A treating chamber is filled with a treating gas containing HCHO, and any sterilization subjects placed in the treating chamber are sterilized. In the sterilization, while maintaining the interior of the treating chamber at given temperature (T1), the treating gas is fed into the treating chamber, and the sterilization subjects are exposed to the treating gas for given period of time (h1). Thereafter, the feeding of treating gas into the treating chamber is discontinued, and while the treating gas remains in the treating chamber, the interior of the treating chamber is heated to thermal treatment temperature (T2) that is higher than the given temperature (T1) but does not exceed the heat resisting temperature (T0) of the sterilization subjects.

## Description

### Technical Field

The present invention relates to technology for the sterilization of various types of objects to be sterilized, including clean rooms and surgeries, surgical tools, medical materials, and nursing care goods, and in particular, relates to a method of sterilization for killing heat-resistant spores at a temperature lower than the actual extinction temperature of the spores (at a temperature at which the biological defense mechanism operates).

### Prior Art

The extinction temperature of heat-resistant spores (simply spores hereafter) is usually 10.0°C or higher, and when sufficient sterilization to spores is to be obtained in a relatively short amount of time, the object to be sterilized must be heated to a high temperature of 120°C or higher. Therefore, an object that is damaged (deformed, degenerated, or similarly impaired) below a temperature of 100°C, such as a plastic product, cannot be subjected to sterilization by heat.

Several methods of sterilization are known for killing spores at a temperature lower than the spore extinction temperature. However, these methods have disadvantages in that the bacterial cell is destroyed by adiabatic expansion of a medium that is forced to invade the bacterial cell by adiabatic compression, or an aqueous bactericide solution is used; therefore, the objects that can be sterilized are limited (Patent References No. 1, No. 2).
Patent Reference 1: JP Unexamined Patent Publication (Kokai) 2004-121161
Patent Reference 2: JP Unexamined Patent Publication (Kokai) 2004-2229

### Disclosure of the Invention

### Problems to Be Solved By the Invention

An object of the present invention is to provide a very versatile method of sterilization and a sterilization apparatus capable of sterilization treatment with strong sterilization power capable of killing spores but without thermally damaging an object to be treated having poor heat resistance, such as a plastic product.

### Means for Solving Problems

In order to solve the above-mentioned problems, the first method of sterilization of the present invention is one wherein a treatment gas containing an active sterilization ingredient is filled inside a sterilization chamber and an object to be sterilized is sterilized inside the treatment chamber. At this time, the treatment gas is fed to the inside of the treatment chamber and the object to be sterilized is exposed to the treatment gas while the inside of the treatment chamber is kept at a pre-determined temperature; then the supply of treatment gas to the inside of the treatment temperature is stopped; and the temperature inside the treatment chamber is raised to as high a heat treatment temperature as possible within a temperature range higher than the pre-determined temperature but not exceeding the upper limit of the heat-resistance temperature of the object to be sterilized.

By means of this method of sterilization, the heat resistance of the spores that are present on the surface and inside the object to be sterilized can be reduced by exposing the object to be sterilized to a treatment gas containing an active sterilization ingredient such as HCHO; therefore, spores can be killed at a temperature that is much lower than the actual extinction temperature by then heating the inside of the treatment chamber. By setting the heat treatment temperature at as high a temperature as possible within a range that does not exceed the upper limit of the heat resistance temperature of the object to be sterilized, it is possible to sterilize with a strong sterilization power capable of killing the spores without thermally damaging the object to be sterilized having poor heat resistance, such as a plastic product. All spores should be eventually killed by heat treatment. Therefore, when HCHO is used as the active sterilization ingredient, for instance, a treatment gas having a low HCHO concentration of 200 ppm or less can be used. Even though the concentration is low, the HCHO itself has a strong permeating force relative to the object to be sterilized and this ingredient is therefore certain to kill spores up to the inside of the object to be sterilized. Moreover, it is possible to sterilize a variety of diverse objects because the mechanism by which a treatment gas is forced to invade the inside of the bacterial cell is not used, nor is an aqueous bactericide solution used.

The second method of sterilization of the present invention is one wherein, after an object to be sterilized has been exposed to a treatment gas containing an active sterilization ingredient, the object to be sterilized is heated to as high a heat treatment temperature as possible within a temperature range higher than the pre-determined temperature but not exceeding the upper limit of the heat-resistance temperature of the object to be sterilized.

By means of this method of sterilization, the heat resistance of the spores that are present on the surface and inside the object to be sterilized can be reduced by exposing the object to be sterilized to a treatment gas containing an active sterilization ingredient such as HCHO; therefore, spores can be killed at a temperature that is much lower than the actual extinction temperature by then heating the inside of the treatment chamber. By setting the heat treatment temperature at as high a temperature as possible within a range that does not exceed the upper limit of the heat resistance temperature of the object to be sterilized, it is possible to sterilize with a strong sterilization power capable of killing the spores without thermally damaging the object to be sterilized having poor heat resistance, such as a plastic product. All spores should be eventually killed by heat treatment. Therefore, when HCHO is used as the active sterilization ingredient, for instance, a treatment gas having a low HCHO concentration of 200 ppm or less can be used. Even though the concentration is low, the HCHO itself has a strong permeating force relative to the object to be sterilized; therefore, this ingredient is certain to kill spores up to the inside of the object to be sterilized. Moreover, it is possible to sterilize a variety of diverse objects because the mechanism by which a treatment gas is forced to invade the inside of the bacterial cell is not used, nor is an aqueous bactericide solution employed.

The third method of sterilization of the present invention is one wherein, after an object to be sterilized has been exposed to an active sterilization ingredient, the object to be sterilized is irradiated with gamma rays or ultraviolet rays.

By means of this method of sterilization, it is possible to reduce the resistance to gamma rays or ultraviolet rays of the spores that are present at the surface and on the inside of an object to be sterilized by exposing the object to be sterilized to a treatment gas containing an active sterilization ingredient such as HCHO; therefore, the spores that are present at the surface and on the inside of the object to be sterilized can be killed by irradiation of the object to be sterilized with gamma rays or ultraviolet rays. Heat treatment is not performed; therefore, the sterilization can be performed with a sterilization power capable of killing the spores without thermally damaging the object to be sterilized having low thermal resistance, such as a plastic product. Moreover, it is possible to sterilize a variety of diverse objects because the mechanism by which a treatment gas is forced to invade the inside of the bacterial cell is not used nor is an aqueous bactericide solution employed.

The first sterilization apparatus of the present invention comprises a treatment chamber for holding an object to be sterilized, an electric heater for heating the treatment chamber, a treatment gas generator for generating treatment gas containing an active sterilization ingredient, a gas conveyance system for introducing the treatment gas generated by the treatment gas generator to inside the treatment chamber, and a control unit; and sterilizes an object to be sterilized inside the treatment chamber when a treatment gas containing an active sterilization ingredient such as HCHO is filled inside this treatment chamber. At this time, the control unit controls the operation of the treatment gas generator and the gas conveyance system and the supply of electricity to the electric heater so that the treatment gas is fed to inside the treatment chamber and the object to be treated is exposed to treatment gas while the inside of the treatment chamber is kept at a pre-determined temperature but; then the supply of treatment gas to inside the treatment temperature is stopped; and the inside of the treatment chamber is heated to as high a heat-treatment temperature as possible within a temperature range higher than the predetermined temperature but not exceeding the upper limit of the heat resistance temperature of the object to be sterilized.

By means of this sterilization apparatus, the heat resistance of the spores that are present on the surface and inside the object to be sterilized can be reduced by exposing the object to be sterilized to a treatment gas containing an active sterilization ingredient such as HCHO; therefore, spores can be killed at a temperature that is much lower than the actual extinction temperature by then heating the inside of the treatment chamber. By setting the heat treatment temperature at as high a temperature as possible within a range that does not exceed the upper limit of the heat resistance temperature of the object to be sterilized, it is possible to sterilize with a strong sterilization power capable of killing the spores without thermally damaging the object to be sterilized having poor heat resistance, such as a plastic product. All spores should be eventually killed by heat treatment. Therefore, when HCHO is used as the active sterilization ingredient, for instance, a treatment gas having a low HCHO concentration of 200 ppm or less can be used. Even though the concentration is low, the HCHO itself has a strong permeating force relative to the object to be sterilized; therefore, this ingredient is certain to kill spores up to the inside of the object to be sterilized. Moreover, it is possible to sterilize a variety of diverse objects because the mechanism by which a treatment gas is forced to invade the inside of the bacterial cell is not used nor is an aqueous bactericide solution employed.

The second sterilization apparatus comprises a treatment chamber for holding an object to be sterilized, a radiation source for irradiation of the object to be sterilized inside the treatment chamber with gamma rays or ultraviolet rays, a treatment gas generator for generating treatment gas containing an active sterilization ingredient, a gas conveyance system for introducing the treatment gas generated by the treatment gas generator to inside the treatment chamber, and a control unit, wherein this control unit controls the operation of the treatment gas generator, the gas conveyance system, and the radiation source so that the treatment gas is fed to inside the treatment chamber and the object to be treated is exposed to treatment gas while the inside of the treatment chamber is kept at a pre-determined temperature, then the supply of treatment gas to inside the treatment temperature is stopped, and the object to be treated is irradiated with gamma rays or ultraviolet rays.

By means of this method of sterilization, it is possible to reduce the gamma-ray or ultraviolet-ray resistance of the spores that are present at the surface and on the inside of an object to be sterilized by exposing the object to be sterilized to a treatment gas containing an active sterilization ingredient such as HCHO; therefore, the spores that are present at the surface and on the inside of the object to be sterilized can be killed by the irradiation of the object to be sterilized with gamma rays or ultraviolet rays. Heat treatment is not performed; therefore, sterilization can be performed with a strong sterilization power capable of killing the spores but without thermally damaging the object to be sterilized that has low thermal resistance, such as a plastic product. Moreover, it is possible to sterilize a variety of diverse objects because the mechanism by which a treatment gas is forced to invade the inside of the bacterial cell is not used, nor is an aqueous bactericide solution employed.

Preferably, 80°C is the maximum heat treatment temperature of the method of sterilization and the sterilization apparatus of the present invention.

Preferably, a treatment gas that contains an active species generated by the oxidation of methanol is the treatment gas of the first and second methods of sterilization and of the first sterilization apparatus.

Once the supply of treatment gas to inside the treatment chamber has been stopped, it is possible to raise the temperature inside the treatment chamber up to a treatment temperature that is higher than the predetermined temperature but lower than the heat resistance temperature of the object to be sterilized with treatment gas remaining inside the treatment chamber of the first method of sterilization and the first sterilization apparatus. By allowing the treatment gas to remain inside the treatment chamber even after the supply of the treatment gas has been stopped, a strong sterilizing effect can be expected due to the synergism of the sterilizing effect of the active sterilization ingredient in the treatment gas and the sterilizing effect of heating.

### Effect of the Invention

By means of the present invention, the heat resistance of the spores present at the surface and inside an object to be sterilized is reduced and then sterilization is performed by raising the treatment temperature to a temperature that is lower than the heat resistance temperature of the object to be treated; therefore, it is possible to perform sterilization treatment with a strong sterilization power capable of killing the spores but without thermally damaging a variety of diverse objects to be sterilized.

By means of the present invention, the resistance of spores present at the surface and inside the object to be sterilized is reduced and then sterilization is performed by irradiation of the object to be sterilized with gamma rays or ultraviolet rays; therefore, it is possible to perform sterilization treatment with a strong sterilization power capable of killing the spores but without thermally damaging a variety of diverse objects to be sterilized.

### Brief Description of the Drawings

[Figure 1] is an oblique view of the outside of a structural example of the sterilization apparatus for conducting the method of sterilization of the present invention.
[Figure 2] is an oblique view of a conceptual representation of the structure of the sterilization apparatus.
[Figure 3] is a structural drawing of a treatment gas feed device.
[Figure 4] is a structural drawing of a treatment gas generator.
[Figure 5] is a temperature curve graph showing an example of changes in temperature inside an object to be sterilized when sterilization is performed by the method of the present invention.

### List of Reference Numerals

10. Sterilization apparatus
20. Treatment chamber (treatment vessel)
25. Electric heater
30. Treatment gas feed device
40. Treatment gas generator
42. Gasification chamber
43. Catalyst cell
50. Gas conveyance system
51. Suction pump (treatment gas conveyor)
52. Humidity regulator
53. Temperature regulator
54. Exhaust gas treatment unit
55. Evacuation pump (gas evacuation unit)
60. Control unit

### Preferred Embodiments of the Invention

Preferred embodiments for conducting the present invention will now be described while referring to the drawings.

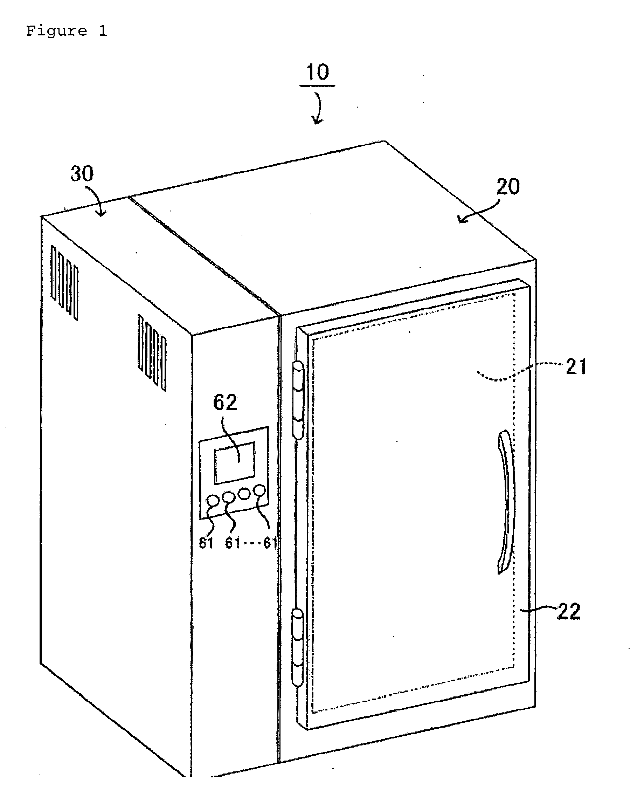

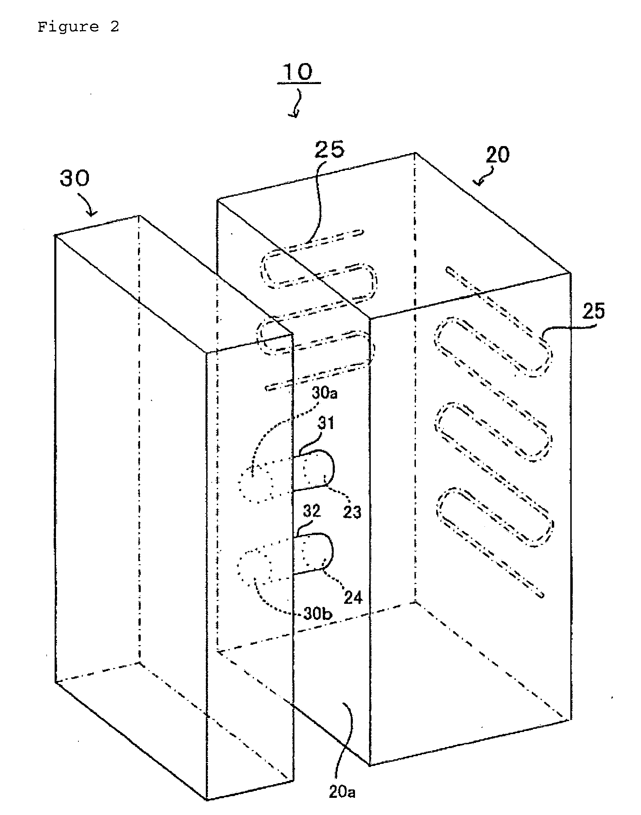

Figure 1 is an oblique view of the outside, showing a structural example of the sterilization apparatus for conducting the method of sterilization of the present invention. Figure 2 is an oblique view of a conceptual representation of the structure of the sterilization apparatus in Figure 1. This sterilization apparatus comprises a treatment chamber 20 for holding an object to be sterilized and a treatment gas feed device 30 for feeding treatment gas to the inside of closed treatment chamber 20.

Treatment chamber 20 is chemical-resistant, heat-resistant and pressure-resistant. An opening 21 for introducing and removing the object to be sterilized is disposed in the front surface of treatment chamber 20. A door 22 is disposed at opening 21 and is designed such that when door 22 is closed, the inside of treatment chamber 20 is sealed airtight. An inlet 23 for introducing treatment gas and an evacuation port 24 for the evacuation of exhaust gas are disposed inside treatment chamber 20. The descending current end of a treatment gas introduction tube 31 is connected to inlet 23, while the ascending current end of an evacuation tube 32 is connected to evacuation port 24. The ascending current end of treatment gas introduction tube 31 is connected to a treatment gas evacuation port 30a of treatment gas feed unit 30, while the descending end of evacuation tube 32 is connected to an exhaust gas intake 30b of treatment gas feed unit 30.

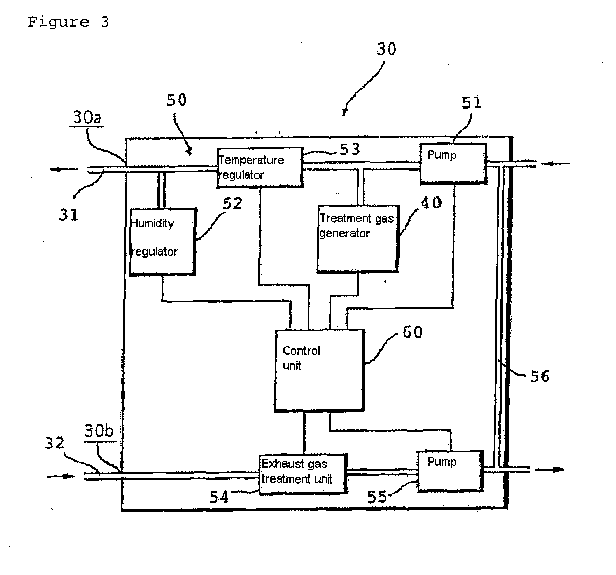

Moreover, an electric heater 25 for heating the inside of treatment chamber 20 is disposed on the inside surface of treatment chamber 20. A temperature sensor, which is not illustrated, is disposed inside treatment chamber 20, and the output of this temperature sensor is input to a control unit 60 (refer to Figure 3) inside treatment gas feed device 30.

Moreover, various switches 61 for setting the treatment gas concentration, temperature, humidity, and supply time h1, the temperature inside treatment chamber 20 (the temperature when treatment gas is fed (pre-determined temperature T1), the heat treatment temperature T2), and similar parameters, as well as a status display unit 62 are disposed at the front surface of treatment gas feed unit 30.

Figure 3 is a structural drawing of treatment gas feed unit 30. Treatment gas feed unit 30 comprises a treatment gas generator 40, a gas conveyance system 50 for introducing treatment gas generated by treatment gas generator 40 to inside sealed treatment chamber 20, and control unit 60. Control unit 60 has both a function for controlling the operation of treatment gas generator 40 and a function for controlling electricity sent to electric heater 25 of treatment chamber 20.

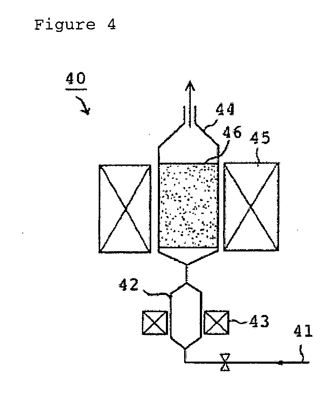

Figure 4 is a structural drawing of treatment gas generator 40. A gasification chamber 42 for feeding methanol from a methanol feed source, which is not illustrated, through a conveyance tube 41, a first temperature regulator 43 for heating gasification chamber 42 from around the outside of the chamber, a substantially cylindrical catalyst cell 44 connected to the top of gasification chamber 42, and a second temperature regulator 45 for heating catalyst cell 44 from around the outside of the cell are disposed inside treatment gas generator 40. A catalyst 46 that has been converted to particulate form is packed inside catalyst cell 44. Platinum, copper, aluminum, carbon, or a mixture thereof is used as catalyst 46.

A predetermined amount of methanol is first fed to gasification chamber 42 when treatment gas is to be generated by treatment gas generator 40. Methanol fed to gasification chamber 42 is gasified by heating and fed to catalyst cell 44. Treatment gas containing HCHO and various types of seed radicals is generated as a result of exposing the methanol gas inside the catalyst cell to a catalyst. The amount of treatment gas generated depends on the amount of methanol gasification in gasification chamber 42, the amount of methanol gas fed to catalyst cell 44, the heating temperature of catalyst cell 44, and similar conditions.

Gas conveyance system 50 has a suction pump (treatment gas conveyor) 51 for feeding outside air or treatment gas (treatment gas or a mixture of treatment gas and outside air) to the inside of treatment chamber 20, a humidity regulator 52 for adjusting the humidity of the treatment gas fed to inside treatment chamber 20, a temperature regulator 53 for adjusting the temperature of the treatment gas fed to inside treatment chamber 20, an exhaust gas treatment unit 54 for treatment (deactivation) of exhaust gas from inside treatment chamber 20, and an evacuation pump (gas evacuation unit) 55 for evacuating the treated exhaust gas. The exhaust port-side path of evacuation pump 55 and the intake-side path of suction pump 51 are connected by a reflux air path 56.

Treatment gas generator 40, suction pump 51, humidity regulator 52, temperature regulator 53, exhaust gas treatment unit 54, and evacuation pump 55 are controlled by control unit 60.

Control unit 60 controls the concentration (amount generated) of treatment gas to a predetermined concentration range by controlling treatment gas generator 40 as it controls the temperature and humidity of the treatment gas fed to inside treatment chamber 20 by controlling humidity regulator 52 and temperature regulator 53, and also controls the amount of treatment gas fed to inside treatment chamber 20 and the amount of exhaust gas emitted from inside treatment chamber 20 by controlling suction pump 51 and evacuation pump 55.

Moreover, control unit 60 controls the amount of current introduced to electric heater 25 in accordance with the detection values of the temperature sensor inside treatment chamber 20.

The method of sterilization by sterilization apparatus 10 designed as described above will now be described.

A specially-trained worker handles the object to be treated, treatment chamber 20, and treatment gas feed device 30 at the time of sterilization.

When sterilization is performed, the object to be sterilized is housed inside treatment chamber 20 with door 22 closed. Then a temperature T1 at the time treatment gas is fed (selected from a range of 20 to 40°C), a heat treatment temperature T2 (T1<T2<T0 ≦ 80°C; T0 is the heat-resistance temperature of the object to be treated), and humidity (selected from within a range of a relative humidity of 70 to 90%), and other parameters are set and sterilization apparatus 10 is turned to the sterilization treatment mode. As a result, electricity is sent to electric heater 25 and the temperature inside treatment chamber 20 rises to the pre-determined temperature T1. Once the inside of treatment chamber 20 becomes temperature T1, treatment gas is fed to inside treatment chamber 20 and the object to be sterilized housed inside treatment chamber 20 is sterilized. In this case, the temperature of the treatment gas fed to the inside of treatment chamber 20 is controlled at a pre-determined temperature T1 that is the same as the temperature inside treatment chamber 20. Moreover, the humidity of the treatment gas that is supplied is controlled to the set humidity H1.

Once a pre-determined time h1 has passed after starting the feed of treatment gas, the supply of treatment gas is stopped. At the same time, the amount of electricity sent to electric heater 25 is increased and the inside of treatment chamber 20 is heated until it reaches the heat treatment temperature T2. Treatment gas remains inside treatment chamber 20 during this time.

When the inside of treatment chamber 20 reaches temperature T2, the electricity to electric heater 25 is cut off. In essence, suction pump 51 and exhaust pump 55 are turned on when treatment gas generator 40 is turned off. Moreover, the exhaust gas from inside treatment chamber 20 is treated by exhaust gas treatment unit 54. The exhaust gas treated by exhaust gas treatment unit 54 is fed to suction pump 51 through reflux air path 56 and air inside treatment chamber 20 is recycled by being fed once again to the inside of treatment chamber 20. This treatment is performed until the concentration of treatment gas inside treatment chamber 20 becomes lower than a pre-determined concentration. Ventilation is performed whereby outside air is introduced to treatment chamber 20.

Once the above-mentioned series of steps is completed, the operator removes the sterilized object from inside treatment chamber 20.

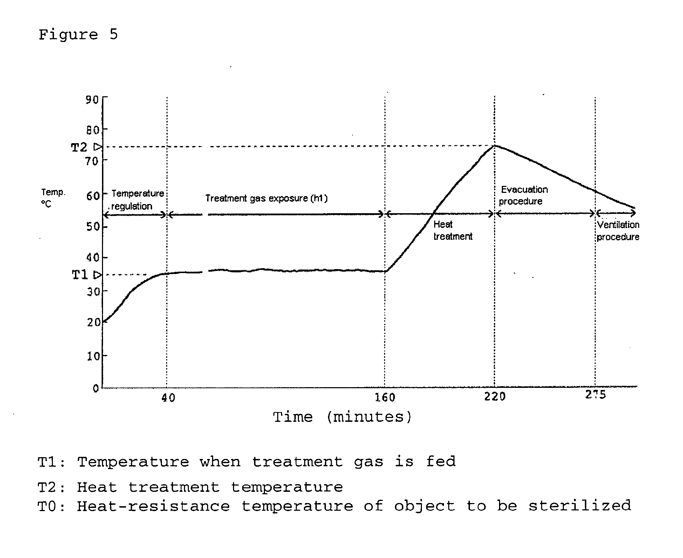

Figure 5 shows the changes in temperature inside the object to be sterilized when sterilization has been performed with temperature T1 when treatment gas is fed set at 35°C and heat treatment temperature T1* set at 75°C. The outside air temperature is approximately 20°C in this example and the temperature inside treatment chamber 20 is therefore adjusted by electric heater 25 before the treatment gas is fed, but when the outside air temperature is 30°C, the temperature can be adjusted by feeding the warm air generated by treatment gas generator 40 to inside treatment chamber 20 and the supply of treatment gas can be started without adjusting the temperature inside treatment chamber 20.
* sic; T2?-Trans. Note.

By means of this method of sterilization, the treatment gas containing HCHO is fed to inside treatment chamber 20 while the inside of treatment chamber 20 is kept at a pre-determined temperature T1 and the object to be sterilized housed inside treatment chamber 20 is exposed to the treatment gas for a predetermined time in order to reduce the heat resistance of the spores present at the surface and inside the object being sterilized; therefore, it is possible to kill all bacteria, including the spores present at the surface and inside the object to be sterilized, below temperature T2, which is much lower than the intrinsic extinction temperature, by heating to heat treatment temperature T2, which is higher than temperature T1 when treatment gas is fed to inside treatment chamber 20, but lower than the heat-resistance temperature T0 of the object to be sterilized. When heat treatment temperature T2 is lower than the heat-resistance temperature of the object to be sterilized, sterilization can be performed with a strong sterilization power capable of killing the spores without thermally damaging even those objects to be sterilized having a low heat resistance, such as a plastic product. Moreover, the treatment gas will remain in treatment chamber 20, even after the supply of treatment gas is stopped; therefore, a strong sterilizing effect is obtained through the synergism of the sterilizing effect of the HCHO contained in the treatment gas and the sterilizing effect of heating. All spores should be eventually killed by heat treatment. Therefore, when HCHO is used as the active sterilization ingredient, for instance, a treatment gas having a low HCHO concentration of 200 ppm or less can be used. Even though the concentration is low, the HCHO itself has a strong permeating force relative to the object to be sterilized and this ingredient is therefore certain to kill spores up to the inside of the object to be sterilized. Moreover, it is possible to sterilize a variety of diverse objects because the mechanism by which a treatment gas is forced to invade the inside of the bacterial cell is not used, nor is an aqueous bactericide solution employed.

Moreover, recognizing the necessity of obtaining a sufficient sterilizing effect by exposure to treatment gas alone, conventional sterilization using a treatment gas containing HCHO involves using a treatment gas with a high HCHO concentration of 2,000 ppm or higher, and there are therefore problems with residual HCHO after sterilization; however, by means of the sterilization method of the present invention, it is possible to use a treatment gas with a very low HCHO concentration of 150 to 200 ppm or less and it is therefore possible to safely sterilize without the problem of residual HCHO.

It should be noted that sterilization apparatus 10 of the above-mentioned embodiment comprises treatment gas generator 40 for generating treatment gas containing HCHO by exposing methanol to a catalyst, but it is also possible to use in its place a sterilization apparatus comprising a treatment gas generator for generating treatment gas by exposing methanol to ultrasonic waves, ultraviolet light, or a plasma. Moreover, it is also possible to use a treatment gas generator that generates treatment gas by heating an aqueous formaldehyde solution or paraformaldehyde (solid) to the gasification temperature or higher, or by exposing these to ultrasonic waves, ultraviolet radiation, a plasma, or high-frequency electromagnetic waves.

The above-mentioned embodiment described an example wherein gas conveyance system 50 of treatment gas feed unit 30 comprised two pumps 51 and 55, but it can also comprise only one pump and be designed such that the supply and emission of gas to and from the inside of the treatment vessel is carried out by this pump.

The above-mentioned embodiment describes a method of sterilization wherein a treatment gas containing HCHO is used as the active sterilization ingredient, but a sufficient sterilizing activity is obtained when a treatment gas is used that contains hydrogen peroxide, ozone, or another active sterilization ingredient in place of HCHO.

The above-mentioned embodiment describes a method of sterilization and a sterilization apparatus wherein heat treatment is performed after the object to be sterilized has been exposed to a treatment gas containing an active sterilization ingredient such as HCHO, but it is also possible to effectively sterilize the object to be sterilized by exposing the object to gamma rays or ultraviolet rays in place of heat treatment.

A sterilization apparatus for feeding this treatment gas to inside this treatment chamber and exposing the object to be sterilized to this treatment gas while keeping the inside of the treatment chamber at a pre-determined temperature; then stopping the supply of this treatment gas to inside this treatment chamber; and irradiating this object to be sterilized with gamma or ultraviolet radiation can be realized by, for instance, adding a radiation source for subjecting the object to be sterilized inside treatment chamber 20 to irradiation with gamma rays or ultraviolet rays to sterilization apparatus 10 of the above-mentioned embodiment and providing control unit 60 or a similar unit with a function for controlling the radiation source. In this case, electric heater 25 is not necessary, but if electric heater 25 is provided, it can be used in combination with treatment by exposure of the object to be sterilized to treatment gas; treatment by heating of the object to be sterilized; or treatment by irradiation of the object to be sterilized with gamma rays or ultraviolet rays.

When treatment by exposure of the object to be sterilized to treatment gas and treatment by irradiation of an object to be sterilized with gamma rays or ultraviolet rays are used in combination with one another, it is possible to more effectively reduce the heat resistance of spores present at the surface and inside an object to be sterilized; therefore, it is possible to use a treatment gas having a lower concentration of active ingredient. The timing by which the object to be sterilized is irradiated with gamma rays or ultraviolet rays can be before heat treatment is started or after the object to be sterilized has been exposed to treatment gas. It is also possible to perform irradiation with gamma or ultraviolet radiation simultaneous to treatment by exposing the object to be sterilized to treatment gas.

### Working Examples

### [Working Examples]

*Bacillus subtilis* (spores) ATCC9372 produced by Cell Tech Laboratory were cultured in agar medium to create a biological indicator (BI) with a live bacteria count of 2.19 x 10⁶/disc, this indicator was buried in a bed mattress, and the mattress was subjected to sterilization by generating MR gas (treatment gas containing radicals attributed to methanol or formaldehyde) and HCHO gas using a small MR gas sterilization apparatus made by Bio Media Co., Ltd. The treatment gas exposure time was 30 minutes and the heat treatment time was 60 minutes in this case. The temperature, humidity, and HCHO gas concentration inside the apparatus when the MR gas was generated and when the HCHO gas was generated are shown below.
When MR gas was generated:
Temperature: 36.0 to 40.09°C, humidity: 80.0 to 88.0%
HCHO gas concentration: 150 ppm When HCHO gas was generated:
Temperature: 37.5 to 40.09°C, humidity: 82.2 to 86.2%
HCHO gas concentration: 200 ppm
The number of live bacteria in the BI was counted after exposure to the treatment gas, and the number of live bacteria in the BI was re-counted after heat treatment. The results are shown in the following Table 1. Moreover, the BI culturing effect for the 7 days after culturing is shown in Table 2.

**[Table 1]**

| | Gas exposure alone | 60°C heat treatment | 80° heat treatment |
|---|---|---|---|
| Object BI (non-gas exposure) | 2.19 x 10⁶ bacteria/Disc | 1.0 x 10⁶ bacteria/Disc | 0.7 x 10⁶ bacteria/Disc |
| MR gas | 1.32 x 10⁵ bacteria/Disc | 0.80 x 10³ bacteria/Disc | 0 bacteria |
| HCHO gas | 2.20 x 10⁶ bacteria/Disc | 1.02 x 10⁴ bacteria/Disc | 0 bacteria |

**[Table 2]**

| Culture time Sampling No. | 12 hours | 6 days | 7 days |
|---|---|---|---|
| MR gas | (+) | (+) | (+) |
| MR gas exposure + 60°C | (-) | (-) | (-) |
| MR gas exposure + 80°C | (-) | (-) | (-) |
| HCHO gas | (+) | (+) | (+) |
| HCHO gas exposure + 60°C | (-) | (+) | (+) |
| HCHO gas exposure + 80°C | (-) | (-) | (-) |
| Blank medium culture | (-) | (-) | (-) |
| Control (unsterilized culture) | (+) | (+) | (+) |

| | | | |
|---|---|---|---|
| (+): Bacteria were growing. (-): Bacteria were not growing. | | | |

It was confirmed by the test results that strong sterilizing activity on a bacteria-reducing level is obtained by exposing an object to be sterilized to MR gas or HCHO gas and then performing heat treatment. It could be confirmed by these test results that sterilization on a bacteria-reducing level is possible by exposure to MR gas or HCHO gas with a low concentration and for a short time (30 minutes with an HCHO concentration of 200 ppm or less), conditions under which sterilizing activity is not usually obtained. In essence, sterilization is possible at a much lower concentration of ingredient than the concentration that is normally used for sterilization; therefore, it is possible to alleviate the problem of residual treatment gas in the object to be sterilized and the toxicity of the treatment gas itself. Moreover, it is possible to reduce the corrosiveness of the treatment gas relative to an object to be sterilized having metal parts

The reason why a strong sterilizing activity on a bacteria-reducing level is obtained by exposing an object to be treated to MR gas or HCHO gas and then heat treating the object appears to be that: (1) exposure to these treatment gases causes considerable damage to the bacterial spores, there is a reduction in the heat resistance thereof, and the spores are easily killed, and (2) the HCHO remaining in the object to be sterilized after exposure to the treatment gas is activated by heating and thereby has sterilization activity, etc.

### [Comparative Example]

*Bacillus subtilis* (spores) ATCC9372 produced by Cell Tech Laboratory were cultured in agar medium to create multiple BIs with a live bacteria count of 1.02 x 10⁶/Disc, and these were subjected only to heat treatment in an incubator. The heat treatment temperatures were 80°C and 60°C, and the number of live bacteria in the BI after treatment was counted. The results are shown in Table 3.

**[Table 3]**

| Number of BI live bacteria before treatment 1.02 x 10⁶/Disc | | |
|---|---|---|
| | Number of live bacteria after 80°C heat treatment (for 1 hour) | Number of live bacteria after 60°C heat treatment |
| BI | 0.7 x 10⁶/Disc | 1.0 x 10⁶/Disc |

It is clear from these results that continuous heat treatment for a specific time with a heat treatment temperature of 80°C, which is higher than that in the Working Example, kills substantially none of the spores of the BI.

As is clear from the above-mentioned results, exposure to treatment gas and heat treatment both have a weak sterilization power against spores when used alone, and a strong sterilizing effect capable of reducing the number of live bacteria to 10⁻⁵ to 10⁻⁶ is not obtained. In contrast to this, a strong sterilizing effect is obtained by exposure to treatment gas and then heat treatment, even if the heat treatment temperature is below 80°C. The reason for this is that although they are not killed, the spores are damaged by exposure to the treatment gas and when heat treated in this weakened state, the proteins of the bacterial cell are easily subjected to thermal denaturation.

### Industrial Applicability

By means of the present invention, it is possible to sterilize various types of objects to be sterilized, including clean rooms and surgeries, surgical tools, medical materials, and nursing care goods, with a strong sterilization power capable of killing spores without thermally damaging the object to be sterilized. Therefore, the method of sterilization and the sterilization apparatus of the present invention can be effectively used for treatment involving virus genome deactivation.

## Claims

1. A method of sterilization wherein a treatment gas containing an active sterilization ingredient is filled inside a sterilization chamber and an object to be sterilized is sterilized inside the treatment chamber, said method of sterilization **characterized in that**
the treatment gas is fed to the inside of the treatment chamber and the object to be sterilized is exposed to the treatment gas while the inside of the treatment chamber is kept at a pre-determined temperature; then the supply of treatment gas to the inside of the treatment chamber is stopped and the temperature inside the treatment chamber is raised up to as high a heat treatment temperature as possible within a temperature range higher than the pre-determined temperature but not exceeding the upper limit of the heat resistance temperature of the object to be sterilized.

2. A method of sterilization, **characterized in that** after an object to be sterilized has been exposed to a treatment gas containing an active sterilization ingredient, the object to be sterilized is heated to as high a heat treatment temperature as possible within a temperature range higher than the pre-determined temperature but not exceeding the upper limit of the heat-resistance temperature of the object to be sterilized.

3. A method of sterilization, **characterized in that** after an object to be sterilized has been exposed to an active sterilization ingredient, the object to be sterilized is irradiated with gamma rays or ultraviolet rays.

4. The method of sterilization according to claim 1, wherein the heat treatment temperature is a maximum of 80°C.

5. A sterilization apparatus, **characterized in that** it comprises
a treatment chamber for holding an object to be sterilized,
an electric heater for heating the treatment chamber,
a treatment gas generator for generating a treatment gas containing an active sterilization ingredient,
a gas conveyance system for introducing the treatment gas generated by the treatment gas generator to inside the treatment chamber, and
a control unit,
wherein this control unit controls the operation of the treatment gas generator and the gas conveyance system and the supply of electricity to the electric heater so that the treatment gas is fed to inside the treatment chamber and the object to be treated is exposed to treatment gas while the inside of the treatment chamber is kept at a pre-determined temperature, then the supply of treatment gas to inside the treatment chamber is stopped, and the inside of the treatment chamber is heated to as high a heat-treatment temperature as possible within a temperature range higher than the pre-determined temperature but not exceeding the upper limit of the heat-resistance temperature of the object to be sterilized.

6. A sterilization apparatus, **characterized in that** it comprises
a treatment chamber for holding an object to be sterilized,
a radiation source for irradiation of the object to be sterilized inside the treatment chamber with gamma rays or ultraviolet rays,
a treatment gas generator for generating a treatment gas containing an active sterilization ingredient,
a gas conveyance system for introducing the treatment gas generated by the treatment gas generator to inside the treatment chamber, and
a control unit,
wherein this control unit controls the operation of the treatment gas generator, the gas conveyance system, and the radiation source so that the treatment gas is fed to inside the treatment chamber and the object to be treated is exposed to treatment gas while the inside of the treatment chamber is kept at a pre-determined temperature, then the supply of treatment gas to inside the treatment chamber is stopped, and the object to be treated is irradiated with gamma rays or ultraviolet rays.

7. The sterilization apparatus according to claim 3, wherein the heat-treatment temperature is a maximum of 80°C.
